# EUROPÄISCHE PATENTSCHRIFT

(11) **EP 2 794 625 B1**
(45) Veröffentlichungstag und Bekanntmachung des Hinweises auf die Patenterteilung: **10.08.2016**
(21) Anmeldenummer: 12830896.2
(22) Anmeldetag: 18.12.2012
(51) Int. Cl.: C07H 3/06, C08B 37/00, C11D 1/66

(54) **VERFAHREN ZUR SÄUREKATALYSIERTEN OLIGOMERISATION VON MONO- ODER DISACCHARIDEN**
METHOD FOR ACID CATALYZED OLIGOMERISATION OF MONOSACCHARIDES OR DISACCHARIDES
PROCÉDÉ D'OLIGOMÉRISATION À CATALYSE ACIDE DE MONO- OU DISACCHARIDES

(30) Priorität: 20.12.2011 DE 102011056679
(43) Veröffentlichungstag der Anmeldung: 29.10.2014
(73) Patentinhaber: Studiengesellschaft Kohle MbH, 45470 Mülheim an der Ruhr (DE)
(72) Erfinder: SCHÜTH, Ferdi, 45470 Mülheim an der Ruhr (DE); RINALDI, Roberto, 45468 Mülheim an der Ruhr (DE); MEINE, Niklas, 40223 Düsseldorf (DE)
(86) Internationale Anmeldenummer: PCT/DE2012/100386
(87) Internationale Veröffentlichungsnummer: WO 2013/091621

(56) Entgegenhaltungen:
- EP-A2- 0 435 657
- EP-A2- 1 284 456
- WO-A1-2005/000905
- US-A1- 2005 000 030

## Beschreibung

Die vorliegende Erfindung betrifft ein Verfahren zur säurekatalysierten Oligomerisation von Mono- oder Disacchariden, bei dem Mono- oder Disaccharide oder Mischungen davon in Gegenwart einer Säure oder Mischungen davon unter Einwirkung von mechanischer Energie unter Bildung von Oligosacchariden in Kontakt gebracht werden.

Im Stand der Technik sind verschiedene Verfahren bekannt, bei denen Saccharide der Einwirkung von mechanischer Energie unterworfen werden.

So wurde bereits zu Beginn des 20. Jahrhunderts versucht, Cellulose durch mechanisches Vermahlen in kleinere Moleküle umzuwandeln. Kugelmühlen wurden eingesetzt, um die Kristallinität der Cellulose zu reduzieren. Grohn et al. (Journal of Polymer Science 1958, 551) entwickelten ein Verfahren zum Umwandeln von Cellulose in wasserlösliche Produkte mit einer Umwandlungsrate von 90 %, bei dem die Cellulose 900 Stunden in einem Stahlkessel vermahlen wurde.

Ein Verfahren zur Herstellung von Galacto-Oligosacchariden, welches im Erhitzen von trockener, pulvriger Lactose bei einer Temperatur von 100 bis 200°C für eine Zeit von 0,5 bis 3 Stunden in Gegenwart einer katalytischen Menge einer anorganischen Säure, ausgewählt aus Salz-, Salpeter- und Schwefelsäure, besteht, ist in der EP 435.657 A1 beschrieben. Aufgrund der Reaktionsbedingungen ist das Verfahren nicht wirtschaftlich.

Ein weiteres Verfahren ist in der WO2004/031244 A1 beschrieben. Diese Druckschrift betrifft Verfahren zur Umgestaltung von Monosacchariden und/oder Oligosacchariden und/oder Polysacchariden mit einem weiteren Monosaccharid, Oligosaccharid oder Polysaccharid und gegebenenfalls mit weiteren Stoffen wie Alkohol, unter kondensierenden Bedingungen, vorzugsweise in säurekatalysierten Reaktionslösungen.

Ein Verfahrensansatz, Cellulose katalytisch zu hydrolysieren, ist in der WO 2009/061750 offenbart, bei dem ein Verfahren zur Herstellung von löslichen Zuckern aus einem Cellulose-haltigen Material beschrieben ist.

Ein weiteres Verfahren ist in der nicht vorveröffentlichten DE 10 2010 052 609 beschrieben, bei dem Cellulose in Gegenwart einer anorganischen und/oder organischen Säure einer mechanischen Behandlung unterworfen wird.

Ebenfalls wurden Mono- und Disaccharide der Einwirkung mechanischer Energie unterworfen. Steurer et al. (Zeitschrift für Physikalische Chemie, 1944, 193, 248-257) behandelten Glukose und Sucrose in einer Schwingmühle, doch selbst nach 100 Stunden mechanischer Behandlung konnte keine Umwandlung in andere Verbindungen festgestellt werden.

Seitens der Erfinder wurde überraschenderweise gefunden, dass die katalytische Umwandlung von Mono- oder Disacchariden oder Mischungen davon in Gegenwart einer anorganischen und/oder organischen Säure unter Einwirkung mechanischer Energie zur Bildung von Oligosacchariden führt. Seitens der Erfinder konnten dabei Oligosaccharide mit einer Anzahl von mehr als zwei bis zu sechs Einheiten eines Monosaccharids hergestellt werden. Die Oligosaccharide sind dabei besonders aus einer Art Monosaccharid gebildet, obgleich auch verschiedene Monosaccharide in der Kette denkbar sind.

Das Di- oder Oligosaccharid enthält vorzugsweise Aldoseeinheiten, bevorzugt eine Aldopentose wie Xylose, Arabinose, und/oder Ribose, und/oder eine Aldohexose, wie Glucose, Galactose und/oder Mannose.

Zur Durchführung des erfindungsgemäßen Verfahrens kann eine anorganische und/oder organische Säure sowie Mischungen davon verwendet werden.

Wenn bei der Durchführung des erfindungsgemäßen Verfahrens eine organische Säure eingesetzt wird, können besonders gute Umwandlungsergebnisse erhalten werden, wenn die organische Säure einen pKs-Wert < 3, besonders einen pKs-Wert von -5 bis 2 aufweist. Geeignete Beispiele sind Benzolsulfonsäure, p-Toluolsulfonsäure, Nitrobenzolsulfonsäuren, 2,4,6-Trimethylbenzolsulfonsäure sowie Derivate der Benzoesäure, Methansulfonsäure, Halogenalkancarbonsäuren wie Trifluoressigsäure, Maleinsäure, Oxalsäure und beliebige Gemische der voranstehenden organischen Säuren. Die verwendeten Säuren sollten vorzugsweise einen pKs-Wert kleiner 2 aufweisen. Bevorzugt sind Säuren mit einem pKs-Wert kleiner als -2.

Bei der Durchführung des erfindungsgemäßen Verfahrens werden gute Umwandlungsergebnisse auch erhalten, wenn eine anorganische Säure mit einem pKs-Wert < 3 eingesetzt wird. Vorzugsweise liegt der pKs-Wert zwischen -14 und 2. Geeignete Beispiele für anorganische Säuren sind Mineralsäuren wie Schwefelsäure, Salzsäure, Phosphorsäure, Phosphorwolframsäure und Salpetersäure, wobei Salpetersäure weniger bevorzugt ist. Es können auch Gemische der voranstehenden Säuren eingesetzt werden. Bevorzugt sind Säuren mit einem pKs-Wert kleiner als -2.

Die anorganische und/oder organische Säure wird im erfindungsgemäßen Verfahren in katalytischen Mengen eingesetzt. Vorzugsweise wird die anorganische und/oder organische Säure in einer Menge von 0,01 bis 10 mmol pro g Mono- oder Disaccharid eingesetzt.

Zur Durchführung des erfindungsgemäßen Verfahrens wird die anorganische und/oder organische Säure nach Art einer "trocken" katalysierten Reaktion mit dem Mono- oder Disaccharid oder Mischungen davon in Kontakt gebracht, und dann wird die so erhaltene Mischung einer mechanischen Behandlung unterzogen.

Dazu wird die anorganische und/oder organische Säure mit dem Mono- oder Disaccharid oder Mischungen davon so in Kontakt gebracht, daß das Mono- oder Disaccharid in einem ersten Verfahrensschritt mit einer Lösung der anorganischen und/oder organischen Säure in einem geeigneten Lösungsmittel imprägniert wird.

Diese Verfahrensweise hat sich besonders für die anorganischen Säuren als vorteilhaft erwiesen. Dazu wird die Säure vorzugsweise zunächst mit einem geeigneten Lösungsmittel vermischt. Als Lösungsmittel sind alle Lösungsmittel, die die Reaktion nicht negativ beeinflussen, geeignet, wie Wasser und organische Lösungsmittel wie Diethylether, Dichlormethan, Ethanol, Methanol, THF, Aceton und jedes andere polare oder unpolare Lösungsmittel, bei dem die eingesetzte Säure löslich ist, oder das eine gute Vermischung von Mono- und/oder Disaccharid und Säure in einer Dispersion ermöglicht, und welches einen Siedepunkt von 100°C und darunter hat.

Bei diesem Verfahrensschritt kann die Lösung bzw. Dispersion der anorganischen und/oder organischen Säure mit dem Mono- und/oder Disaccharid vermischt und gegebenenfalls für einige Zeit stehen gelassen werden. Vor der mechanischen Behandlung des Mono- und/oder Disaccharids wird das Lösungsmittel wieder entfernt. Insbesondere, wenn als Lösungsmittel ein niedrigsiedendes Lösungsmittel eingesetzt wird, kann dieses auf einfache Weise, entweder durch leichtes Erwärmen und/oder durch Anlegen von Vakuum wieder entfernt werden. Die Säure, die üblicherweise einen höheren Siedepunkt hat, verbleibt auf dem Mono- und/oder Disaccharid.

Anschließend erfolgt die mechanische Behandlung des Mono- und/oder Disaccharids in Gegenwart der anhaftenden anorganischen und/oder organischen Säure. Es wurde festgestellt, dass der Umwandlungsgrad des Mono- und/oder Disaccharids durch das Imprägnieren des Mono- und/oder Disaccharids mit anorganischer und/oder organischer Säure in Gegenwart eines Lösungsmittels gesteigert werden kann.

Die mechanische Behandlung erfolgt durch Vermahlen. Als Mühlen können solche eingesetzt werden, die unter Verwendung von Mahlkörpern das Mahlgut zerkleinern, wie z. B. Schwingmühlen, Rührwerksmühlen, Rührwerkskugelmühlen, Kugelmühlen usw. Besonders bevorzugt sind Kugelmühlen.

Wie bereits eingangs berichtet, können mit dem erfindungsgemäßen Verfahren Umwandlungen der Mono- und/oder Disaccharide zu Oligosacchariden von bis zu 80% erreicht werden. Es werden in der Regel Mischungen von wasserlöslichen Saccharid-Dimeren und -Oligomeren, wie Cellobiose, und Monosaccharid erhalten, wobei die Bildung von Nebenprodukten weitgehend vermieden werden kann.

Wird das erfindungsgemäße Verfahren in einer Kugelmühle durchgeführt, so haben sich Drehzahlen von 400 bis 1.200, vorzugsweise 800 bis 1.000 U/min als geeignet erwiesen. Die Reaktionszeit, d. h. die Zeit, in der die mechanische Behandlung erfolgt, beträgt üblicherweise von 0,01 bis 24 Stunden, wobei Zeiträume von 1,5 bis 12 Stunden ausreichend sind.

Weiterhin ist es möglich, die erhaltenen Oligosaccharide durch Zugabe eines oder mehrerer Fettalkohole zu modifizieren. Hierzu können der Oligomermischung vorzugsweise ein oder mehrere Fettalkohole mit 8 bis 22 Kohlenstoffatomen als aliphatische, langkettige, einwertige, primäre Alkohole in einer Menge bis zu 10 Mol.-% bezogen auf das Mono- oder Disaccharid zugesetzt werden. Die Kohlenwasserstoffreste sind dabei unverzweigt und können auch ein- oder mehrfach ungesättigt sein. Auf diese Weise können modifiizierte Oligosaccharide oder Mischungen davon erhalten werden.

Die erfindungsgemäß erhaltenen Oligosaccharide oder deren durch Fettalkohole modifizierten Derivate können als oberflächenaktive Mittel oder Betonadditive verwendet werden.

Die vorliegende Erfindung wird in den nachfolgenden Beispielen näher erläutert, ohne die Erfindung auf diese Beispiele zu beschränken.

### Beispiele

### Beispiel 1

Ein Gemisch aus 1,00 g D-(+)-Glukose (Handelsprodukt der Firma Aldrich, USA) und 0,175 g para-Toluolsulfonsäure-Monohydrat (Handelsprodukt der Firma Aldrich, USA) wurde in einem Stahlbecher mit Stahlkugeln (5 Stahlkugeln; Einzelgewicht 3,95 g) in einer Pulverisette P7 der Firma Fritsch vermahlen. Die Drehzeit der Hauptscheibe betrug 800 U/min.

Eine Probe des erhaltenen Feststoffs wurde in Wasser gelöst und mittels HPLC-Analyse und GPC-Analyse untersucht. Weiterhin wurde die wässrige Lösung nach Entfernung der Säure mittels Festphasenextraktion (SPE-Kartusche, Chromafix, HR-XA (L), Handelsprodukt der Firma Macherey-Nagel) mit einem Massenspektrometer (ESI-MS, Bruker ESQ 3000; high resolution mass determinations: Bruker APEX III FTMS (7 T magnet)) untersucht.

Die säurekatalysierte Oligomerisation von Glukose in der Kugelmühle ergab innerhalb von 5 Stunden Mahlzeit einen Umsatz der Glukose zu wasserlöslichen Produkten, die aus 63 % Oligosacchariden, 10 % Cellobiose und 26 % Glucose bestehen.

### Beispiel 2

Ein Gemisch aus 1,00 g D-(+)-Cellobiose (Handelsprodukt der Firma Fluka, Schweiz) und 0,175 g para-Toluolsulfonsäure-Monohydrat (Handelsprodukt der Firma Aldrich, USA) wurde in einem Stahlbecher mit Stahlkugeln (5 Stahlkugeln; Einzelgewicht 3,95 g) in einer Pulverisette P7 der Firma Fritsch vermahlen. Die Drehzeit der Hauptscheibe betrug 800 U/min.

Eine Probe des erhaltenen Feststoffs wurde in Wasser gelöst und mittels HPLC-Analyse und GPC-Analyse untersucht. Weiterhin wurde die wässrige Lösung nach Entfernung der Säure mittels Festphasenextraktion (SPE-Kartusche, Chromafix, HR-XA (L), Handelsprodukt der Firma Macherey-Nagel) mit einem Massenspektrometer (ESI-MS, Bruker ESQ 3000; high resolution mass determinations: Bruker APEX III FTMS (7 T magnet)) untersucht.

Die säurekatalysierte Oligomerisation von Cellobiose in der Kugelmühle ergab innerhalb von 5 Stunden Mahlzeit einen Umsatz der Cellobiose zu wasserlöslichen Produkten, die aus 60 % Oligosacchariden, 33 % Cellobiose und 7 % Glucose bestehen.

### Beispiel 3

Ein Gemisch aus 1,00 g D-(+)-Xylose (Handelsprodukt der Firma Fluka, Schweiz) und 0,175 g para-Toluolsulfonsäure-Monohydrat (Handelsprodukt der Firma Aldrich, USA) wurde in einem Stahlbecher mit Stahlkugeln (5 Stahlkugeln; Einzelgewicht 3,95 g) in einer Pulverisette P7 der Firma Fritsch vermahlen. Die Drehzeit der Hauptscheibe betrug 800 U/min.

Eine Probe des erhaltenen Feststoffs wurde in Wasser gelöst und mittels HPLC-Analyse und GPC-Analyse untersucht. Weiterhin wurde die wässrige Lösung nach Entfernung der Säure mittels Festphasenextraktion (SPE-Kartusche, Chromafix, HR-XA (L), Handelsprodukt der Firma Macherey-Nagel) mit einem Massenspektrometer (ESI-MS, Bruker ESQ 3000; high resolution mass determinations: Bruker APEX III FTMS (7 T magnet)) untersucht.

Die säurekatalysierte Oligomerisation von Xylose in der Kugelmühle ergab innerhalb von 5 Stunden Mahlzeit einen Umsatz der Xylose zu wasserlöslichen Produkten, die aus 79 % Oligosacchariden und 21 % Xylose bestehen.

### Beispiel 4

Ein Gemisch aus 1,00 g D-(+)-Glukose (Handelsprodukt der Firma Aldrich, USA) und 0,146 g Benzolsulfonsäure (Handelsprodukt der Firma Aldrich, USA) wurde in einem Stahlbecher mit Stahlkugeln (5 Stahlkugeln; Einzelgewicht 3,95 g) in einer Pulverisette P7 der Firma Fritsch vermahlen. Die Drehzeit der Hauptscheibe betrug 800 U/min.

Eine Probe des erhaltenen Feststoffs wurde in Wasser gelöst und mittels HPLC-Analyse und GPC-Analyse untersucht. Weiterhin wurde die wässrige Lösung nach Entfernung der Säure mittels Festphasenextraktion (SPE-Kartusche, Chromafix, HR-XA (L), Handelsprodukt der Firma Macherey-Nagel) mit einem Massenspektrometer (ESI-MS, Bruker ESQ 3000; high resolution mass determinations: Bruker APEX III FTMS (7 T magnet)) untersucht.

Die säurekatalysierte Oligomerisation von Glukose in der Kugelmühle ergab innerhalb von 5 Stunden Mahlzeit einen Umsatz der Glukose zu wasserlöslichen Produkten, die aus 71 % Oligosacchariden, 12 % Cellobiose und 17 % Glucose bestehen.

### Beispiel 5

Ein Gemisch aus 0,50 g D-(+)-Glukose (Handelsprodukt der Firma Aldrich, USA) und 0,5 g Kaolinit (Handelsprodukt der Firma Fluka, Schweiz) wurde in einem Stahlbecher mit Stahlkugeln (5 Stahlkugeln; Einzelgewicht 3,95 g) in einer Pulverisette P7 der Firma Fritsch vermahlen. Die Drehzeit der Hauptscheibe betrug 800 U/min.

Eine Probe des erhaltenen Feststoffs wurde in Wasser gelöst und mittels HPLC-Analyse und GPC-Analyse untersucht. Weiterhin wurde die wässrige Lösung nach Entfernung der Säure mittels Festphasenextraktion (SPE-Kartusche, Chromafix, HR-XA (L), Handelsprodukt der Firma Macherey-Nagel) mit einem Massenspektrometer (ESI-MS, Bruker ESQ 3000; high resolution mass determinations: Bruker APEX III FTMS (7 T magnet)) untersucht.

Die säurekatalysierte Oligomerisation von Glukose in der Kugelmühle ergab innerhalb von 10 Stunden Mahlzeit einen Umsatz der Glukose zu wasserlöslichen Produkten, die aus 73 % Oligosacchariden, 2 % Disacchariden und 24 % Glucose bestehen.

### Beispiel 6

Ein Gemisch aus 0,50 g D-(+)-Xylose (Handelsprodukt der Firma Fluka, Schweiz) und 0,5 g Kaolinit (Handelsprodukt der Firma Fluka, Schweiz) wurde in einem Stahlbecher mit Stahlkugeln (5 Stahlkugeln; Einzelgewicht 3,95 g) in einer Pulverisette P7 der Firma Fritsch vermahlen. Die Drehzeit der Hauptscheibe betrug 800 U/min.

Eine Probe des erhaltenen Feststoffs wurde in Wasser gelöst und mittels HPLC-Analyse und GPC-Analyse untersucht. Weiterhin wurde die wässrige Lösung nach Entfernung der Säure mittels Festphasenextraktion (SPE-Kartusche, Chromafix, HR-XA (L), Handelsprodukt der Firma Macherey-Nagel) mit einem Massenspektrometer (ESI-MS, Bruker ESQ 3000; high resolution mass determinations: Bruker APEX III FTMS (7 T magnet)) untersucht.

Die säurekatalysierte Oligomerisation von Xylose in der Kugelmühle ergab innerhalb von 10 Stunden Mahlzeit einen Umsatz der Xylose zu wasserlöslichen Produkten, die aus 76 % Oligosacchariden, 3 % Disacchariden und 21 % Xylose bestehen.

### Beispiel 7

Ein Gemisch aus 0,50 g D-(+)-Cellobiose (Handelsprodukt der Firma Fluka, Schweiz) und 0,5 g Kaolinit (Handelsprodukt der Fluka) wurde in einem Stahlbecher mit Stahlkugeln (5 Stahlkugeln; Einzelgewicht 3,95 g) in einer Pulverisette P7 der Firma Fritsch vermahlen. Die Drehzeit der Hauptscheibe betrug 800 U/min.

Eine Probe des erhaltenen Feststoffs wurde in Wasser gelöst und mittels HPLC-Analyse und GPC-Analyse untersucht. Weiterhin wurde die wässrige Lösung nach Entfernung der Säure mittels Festphasenextraktion (SPE-Kartusche, Chromafix, HR-XA (L), Handelsprodukt der Firma Macherey-Nagel) mit einem Massenspektrometer (ESI-MS, Bruker ESQ 3000; high resolution mass determinations: Bruker APEX III FTMS (7 T magnet)) untersucht.

Die säurekatalysierte Oligomerisation von Cellobiose in der Kugelmühle ergab innerhalb von 10 Stunden Mahlzeit einen Umsatz der Cellobiose zu wasserlöslichen Produkten, die aus 70 % Oligosacchariden, 19 % Disacchariden und 4 % Glucose bestehen.

### Beispiel 8

Ein Gemisch aus 0,50 g D-(+)-Cellobiose (Handelsprodukt der Firma Fluka, Schweiz) und 0,5 g Amberlyst15 DRY (Handelsprodukt der Firma Rohm&Haas, DE) wurde in einem Stahlbecher mit Stahlkugeln (5 Stahlkugeln; Einzelgewicht 3,95 g) in einer Pulverisette P7 der Firma Fritsch vermahlen. Die Drehzeit der Hauptscheibe betrug 800 U/min.

Eine Probe des erhaltenen Feststoffs wurde in Wasser gelöst und mittels HPLC-Analyse und GPC-Analyse untersucht. Weiterhin wurde die wässrige Lösung nach Entfernung der Säure mittels Festphasenextraktion (SPE-Kartusche, Chromafix, HR-XA (L), Handelsprodukt der Firma Macherey-Nagel) mit einem Massenspektrometer (ESI-MS, Bruker ESQ 3000; high resolution mass determinations: Bruker APEX III FTMS (7 T magnet)) untersucht.

Die säurekatalysierte Oligomerisation von Cellobiose in der Kugelmühle ergab innerhalb von 5 Stunden Mahlzeit einen Umsatz der Cellobiose zu wasserlöslichen Produkten, die aus 80 % Oligosacchariden, 12 % Cellobiose und 8 % Glucose bestehen.

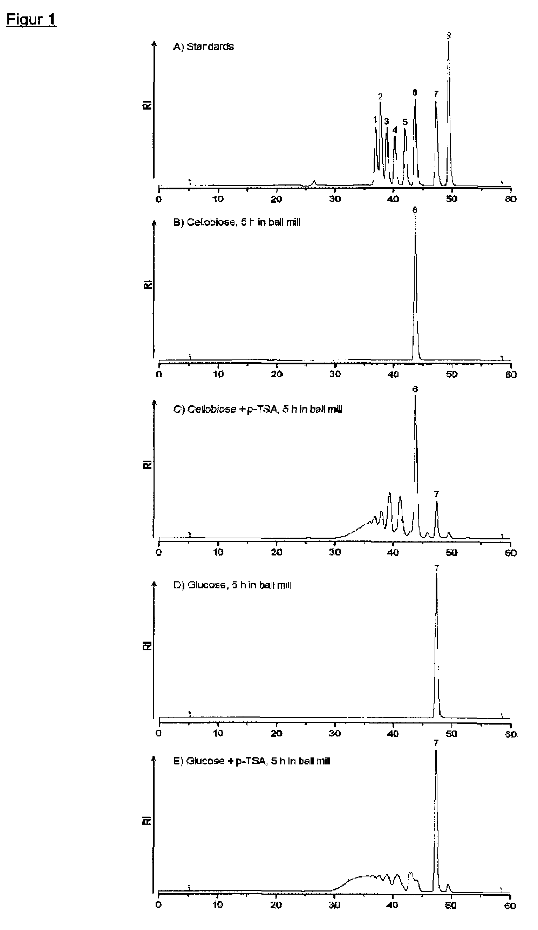

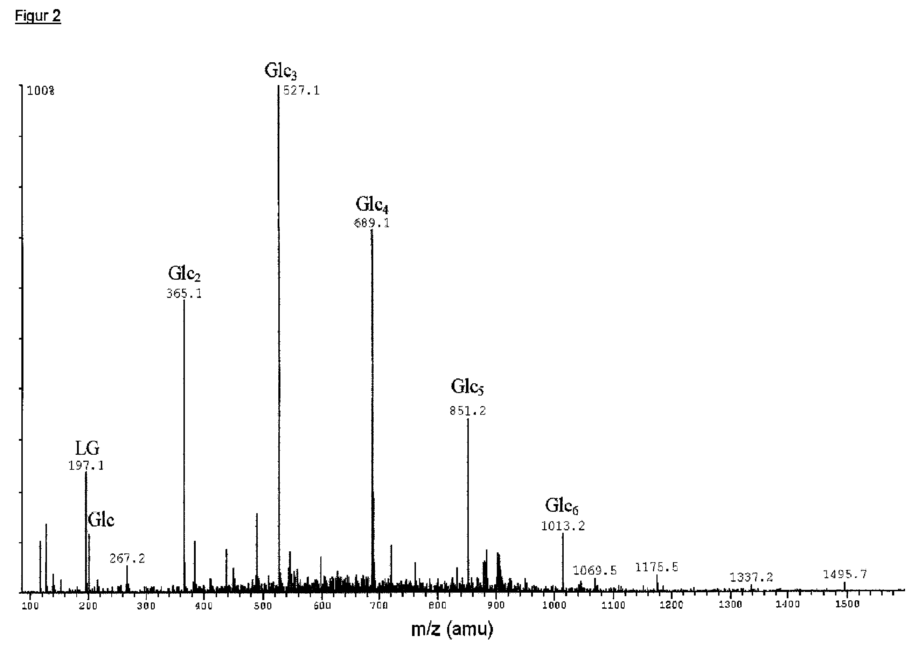

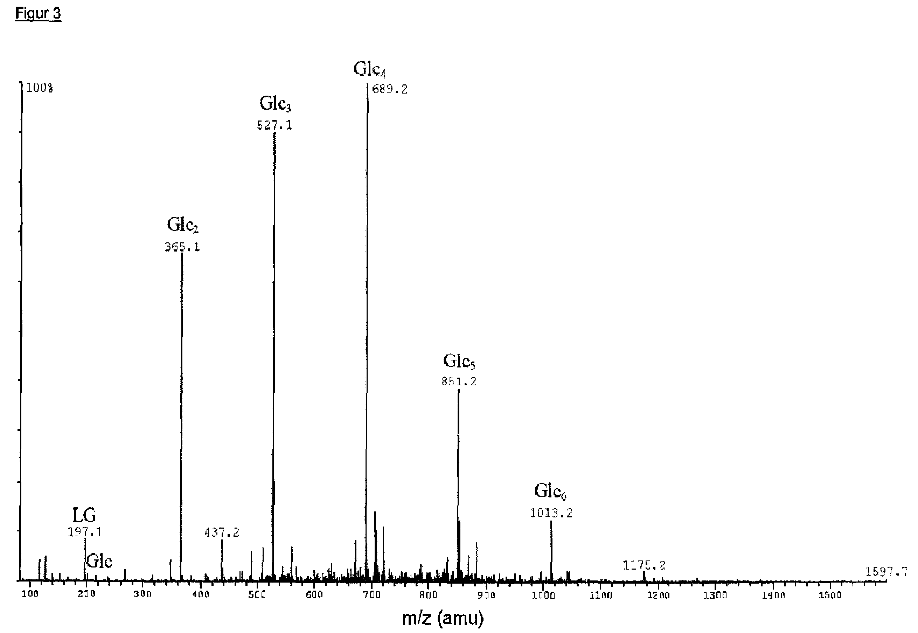

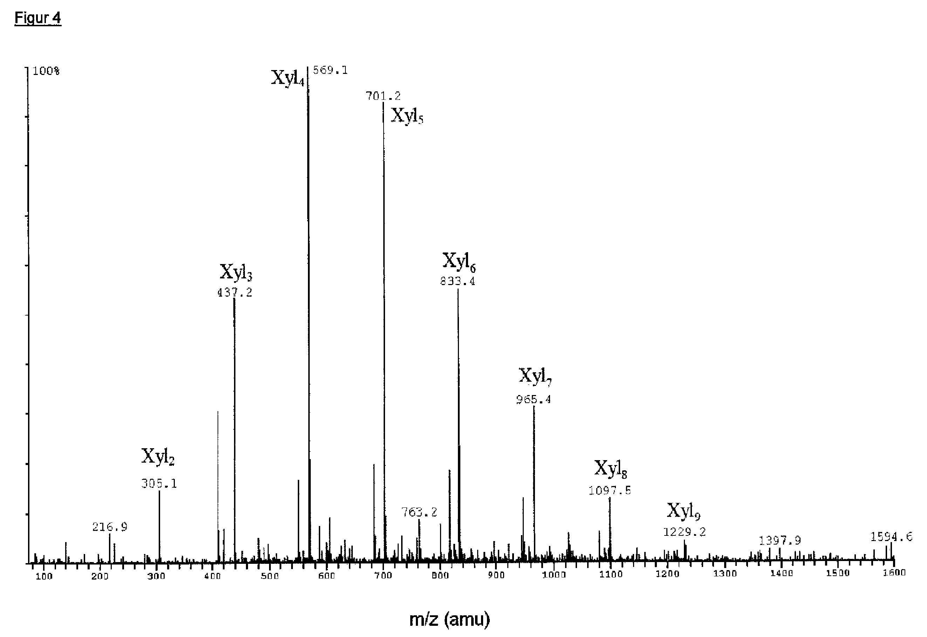

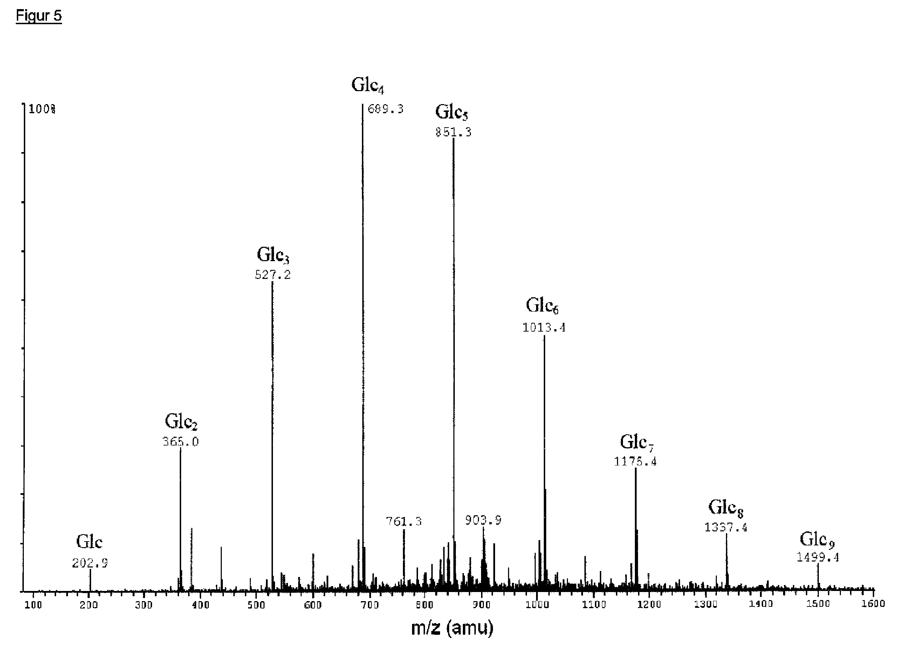

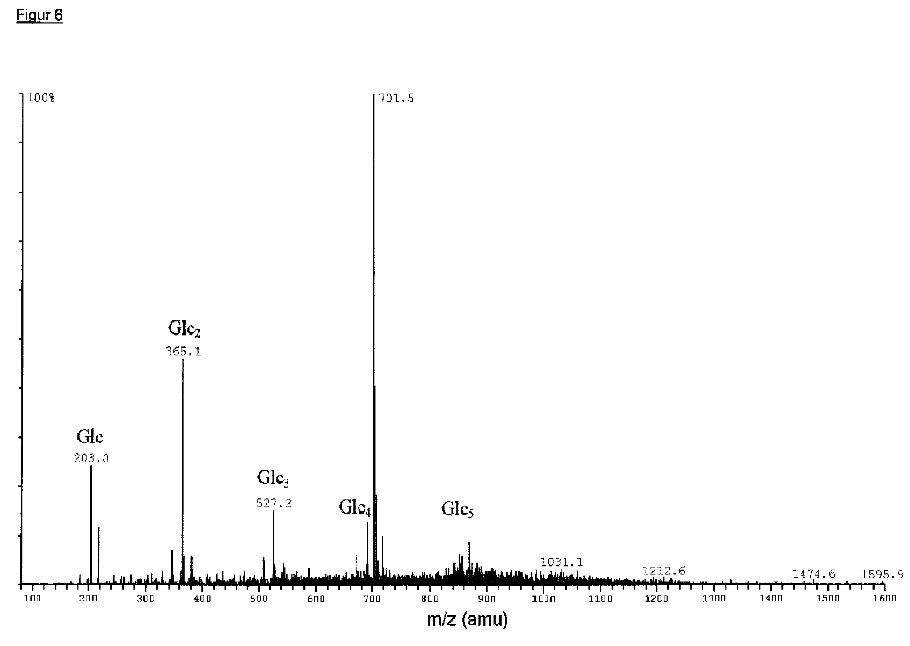

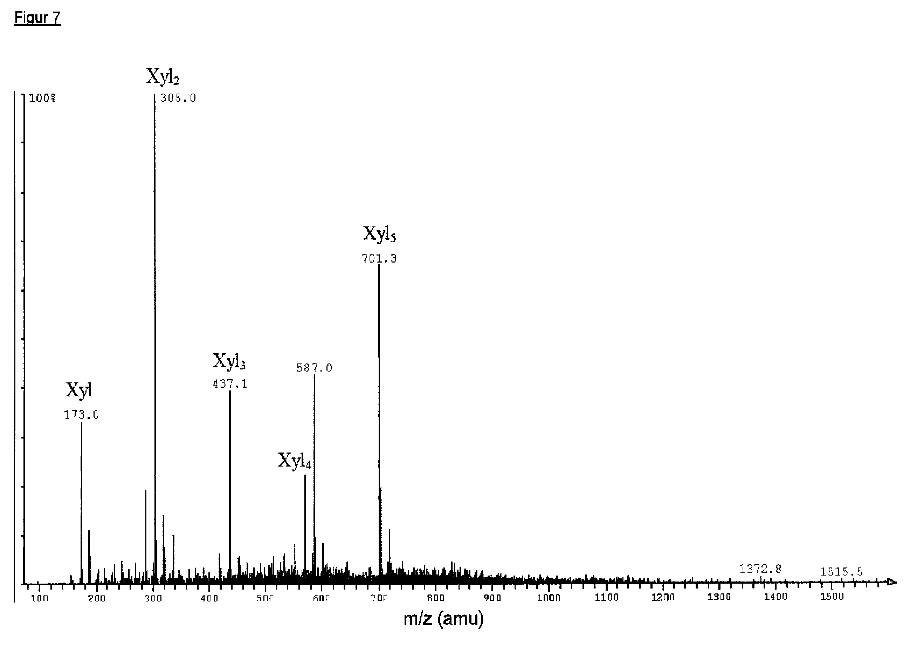

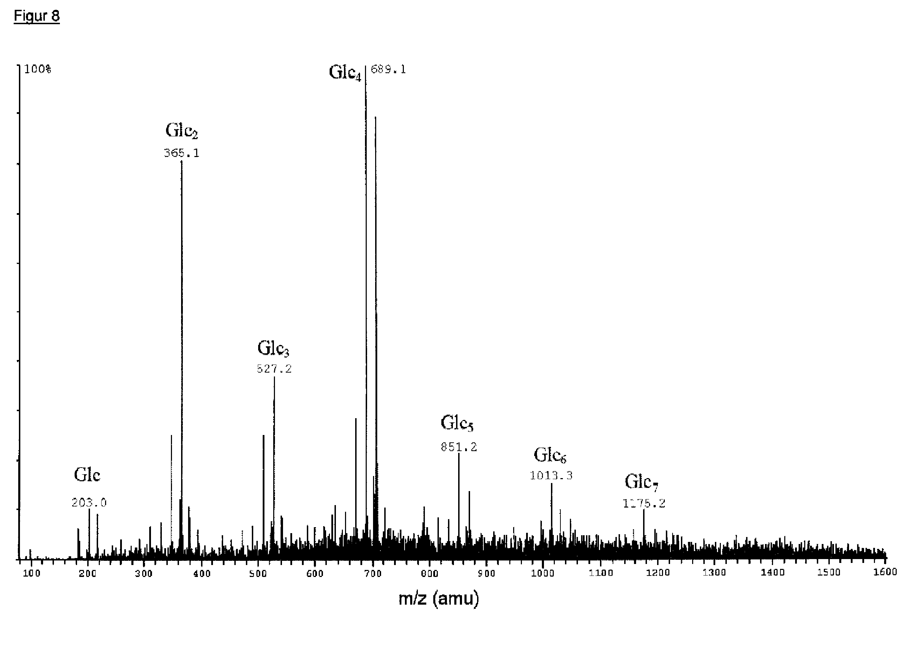

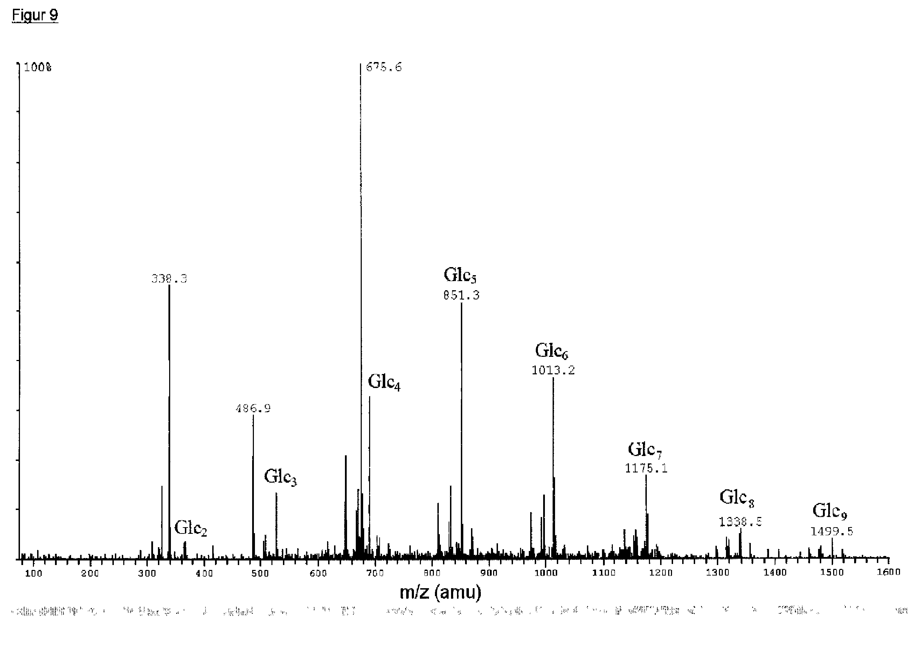

Die chromatographischen und ESI-MS-Untersuchungen der in den Beispielen erhaltenen Reaktionsprodukte sind in den Figuren 1 bis 9 dargestellt. Dabei zeigen:
Figur 1 GPC-Chromatogramme (4 x TSKgel G-Oligo-PW, 7.8 mm ID x 30.0 cm und TSKgel Oligo Guardco; Eluent: Milli-Q® Wasser, flow rate 0.8 mL min⁻¹) von:
   A) mit den Standards: **1** Maltoheptaose, **2** Maltohexaose, **3** Maltopentaose, **4** Maltotetraose, **5** Maltotriose, **6** Cellobiose, **7** Glucose, 8 Glycerin,
   B) Cellobiose 5 h in der Kugelmühle,
   C) Cellobiose + p-TSA 5 h in der Kugelmühle,
   D) Glukose 5 h in der Kugelmühle, E) Glukose + p-TSA 5 h in der Kugelmühle;
Figur 2 ein ESI-MS (pos. Modus) mit einem Spektrum der Oligomerisationsprodukte nach Reaktion von Glukose mit p-TSA in der Kugelmühle. Die m/z-Werte entsprechen den [M+Na]⁺-Ionen. (Glc: Glukosefragment, LG: Levoglukosanfragment);
Figur 3 ein ESI-MS Spektrum (pos. Modus) der Oligomerisationsprodukte nach Reaktion von Cellobiose mit p-TSA in der Kugelmühle. Die m/z-Werte entsprechen den [M+Na]⁺-Ionen. (Glc: Glukosefragment, LG: Levoglukosanfragment);
Figur 4 ein ESI-MS (pos. Modus) mit einem Spektrum der Oligomerisationsprodukte nach Reaktion von Xylose mit p-TSA in der Kugelmühle. Die m/z-Werte entsprechen den [M+Na]⁺-lonen. (Xyl: Xylosefragment);
Figur 5 ein ESI-MS (pos. Modus) mit einem Spektrum der Oligomerisationsprodukte nach Reaktion von Glukose mit BSA in der Kugelmühle. Die m/z-Werte entsprechen den [M+Na]⁺-lonen. (Glc: Glukosefragmente);
Figur 6 ein ESI-MS (pos. Modus) mit einem Spektrum der Oligomerisationsprodukte nach Reaktion von Glukose mit Kaolinit in der Kugelmühle. Die m/z-Werte entsprechen den [M+Na]⁺-lonen. (Glc: Glukosefragmente);
Figur 7 ein ESI-MS (pos. Modus) mit einem Spektrum der Oligomerisationsprodukte nach Reaktion von Xylose mit Kaolinit in der Kugelmühle. Die m/z-Werte entsprechen den [M+Na]⁺-lonen. (Glc: Glukosefragmente);
Figur 8 ein ESI-MS (pos. Modus) mit einem Spektrum der Oligomerisationsprodukte nach Reaktion von Cellobiose mit Kaolinit in der Kugelmühle. Die m/z-Werte entsprechen den [M+Na]⁺-lonen. (Glc: Glukosefragmente), und
Figur 9 ein ESI-MS (pos. Modus) mit einem Spektrum der Oligomerisationsprodukte nach Reaktion von Cellobiose mit Amberlyst15DRY in der Kugelmühle. Die m/z-Werte entsprechen den [M+Na]⁺-lonen. (Glc: Glukosefragmente).

## Patentansprüche

1. Verfahren zur säurekatalysierten Oligomerisation von Mono- und/oder Disacchariden, bei dem ein Mono- und/oder Disaccharid oder Mischungen davon in Gegenwart einer anorganischen und/oder organischen Säure mechanisch behandelt wird, bei dem die Mono- und/oder Disaccharide vor der mechanischen Behandlung mit der Säure oder einem Gemisch davon in einem Lösungsmittel behandelt werden und das Lösungsmittel vor der mechanischen Behandlung entfernt wird, wobei die mechanische Behandlung ein Vermahlen ist.

2. Verfahren nach Anspruch 1, bei dem als Mono- und/oder Disaccharid eine Aldopentose oder Aldohexose, deren Dimere und/oder Mischungen davon eingesetzt werden.

3. Verfahren nach Anspruch 1 oder 2, bei dem das Mono- und/oder Disaccharid in Gegenwart einer anorganischen und/oder organischen Säure in Form einer Pulvermischung oder einer Aufschlämmung mechanisch behandelt wird.

4. Verfahren nach einem der vorhergehenden Ansprüche, bei dem die Säure einen pKs-Wert von -14 bis 2 hat.

5. Verfahren nach einem der vorhergehenden Ansprüche, bei dem die anorganische Säure aus Schwefelsäure, Salzsäure, Phosphorsäure, Salpetersäure, Halogenalkancarbonsäuren wie Trifluoressigsäure, Phosphorwolframsäure und deren beliebigen Gemischen ausgewählt ist.

6. Verfahren nach einem der vorhergehenden Ansprüche, bei dem die organische Säure aus Benzolsulfonsäure, besonders p-Toluolsulfonsäure, Nitrobenzolsulfonsäuren, 2,4,6-Trimethylbenzolsulfonsäure oder Derivaten der voranstehenden, Methansulfonsäure, Maleinsäure, Oxalsäure und deren Gemische ausgewählt ist.

7. Verfahren nach Anspruch 1, **dadurch gekennzeichnet, dass** die Säure aus sauren polymeren oder anorganischen Ionenaustauschern oder sauren anorganischen Metalloxiden ausgewählt ist.

8. Verfahren nach einem der vorhergehenden Ansprüche, bei dem die Säure in einer Menge von 0,01 bis 10 mmol pro g Mono- und/oder Disaccharid eingesetzt wird.

9. Verfahren nach einem der vorhergehenden Ansprüche, bei dem die mechanische Behandlung ein Vermahlen ist, bei der das Mahlgut unter Verwendung von Mahlkörpern zerkleinert wird.

10. Verfahren nach einem der vorhergehenden Ansprüche, bei dem die Mühle aus Schwingmühlen, Rührwerksmühlen, Rührwerkskugelmühlen und Kugelmühlen ausgewählt ist.

11. Verfahren nach einem der vorhergehenden Ansprüche, bei dem das die Reaktionsprodukte enthaltenden Mischung nach der mechanischen Behandlung von der anhaftenden Säure befreit wird und gegebenenfalls in die einzelnen Reaktionsprodukte aufgetrennt wird.

## Claims

1. A method for the acid-catalyzed oligomerization of monosaccharides and/or disaccharides, in which a monosaccharide and/or disaccharide or mixtures thereof is mechanically treated in the presence of an inorganic and/or organic acid, in which the mono- and/or disaccharides are treated before the mechanical treatment with the acid or a mixture thereof in a solvent and in which the solvent is removed before the mechanical treatment in which the mechanical treatment is a grinding.

2. The method as claimed in claim 1, in which the monosaccharide and/or disaccharide used is an aldopentose or aldohexose, dimers thereof and/or mixtures thereof.

3. The method as claimed in claim 1 or 2, in which the monosaccharide and/or disaccharide is mechanically treated in the presence of an inorganic and/or organic acid in the form of a powder mixture or a slurry.

4. The method as claimed in one of the preceding claims, in which the acid has a pKa value of -14 to 2.

5. The method as claimed in one of the preceding claims, in which the inorganic acid is selected from sulfuric acid, hydrochloric acid, phosphoric acid, nitric acid, haloalkanecarboxylic acids such as trifluoroacetic acid, phosphotungstic acid and any desired mixtures thereof.

6. The method as claimed in one of the preceding claims, in which the organic acid is selected from benzenesulfonic acid, particularly p-toluenesulfonic acid, nitrobenzenesulfonic acids, 2,4,6-trimethylbenzenesulfonic acid or derivatives of the above, methanesulfonic acid, maleic acid, oxalic acid and mixtures thereof.

7. The method as claimed in claim 1, **characterized in that** the acid is selected from acidic polymeric or inorganic ion exchangers or acidic inorganic metal oxides.

8. The method as claimed in one of the preceding claims, in which the acid is used in an amount of from 0.01 to 10 mmol per g of monosaccharide and/or disaccharide.

9. The method as claimed in one of the preceding claims, in which the mechanical treatment is a grinding, in which the material to be ground is comminuted using grinding bodies.

10. The method as claimed in one of the preceding claims, in which the mill is selected from vibratory mills, stirred mills, stirred ball mills and ball mills.

11. The method as claimed in one of the preceding claims, in which the mixture comprising the reaction products is freed after the mechanical treatment from the adhering acid and, if desired, is separated into the individual reaction products.

## Revendications

1. Procédé d'oligomérisation à catalyse acide de mono- et/ou disaccharides, consistant à traiter mécaniquement un mono- et/ou disaccharide ou leurs mélanges en présence d'un acide inorganique et/ou organique, lesdits mono- et/ou disaccharides étant traités, préalablement audit traitement mécanique, avec ledit acide ou un mélange de celui-ci dans un solvant, ledit solvant étant enlevé avant le traitement mécanique, ledit traitement mécanique étant un broyage.

2. Procédé selon la revendication 1, dans lequel on met en oeuvre, en tant que mono- et/ou disaccharide, un aldopentose ou aldohexose, leur dimères et/ou mélanges.

3. Procédé selon les revendications 1 ou 2, dans lequel ledit mono- et/ou disaccharide qui est traité mécaniquement en présence d'un acide inorganique et/ou organique, se présente sous forme d'un mélange pulvérulent ou d'une suspension.

4. Procédé selon l'une des revendications précédentes, ledit acide ayant un pKa compris entre -14 et 2.

5. Procédé selon l'une des revendications précédentes, dans lequel ledit acide inorganique est choisi parmi l'acide sulfurique, l'acide chlorhydrique, l'acide phosphorique, l'acide nitrique, les acides carboxyliques de type halogénoalcane tels que l'acide trifluoroacétique, l'acide phosphotungstique et un quelconque mélange de ceux-ci.

6. Procédé selon l'une des revendications précédentes, dans lequel ledit acide organique est choisi parmi l'acide benzènesulfonique, notamment l'acide p-toluènesulfonique, les acides nitrobenzènesulfoniques, l'acide 2,4,6-triméthylbenzènesulfonique ou les dérivés des composés mentionnés ci-avant, l'acide méthanesulfonique, l'acide maléique, acide oxalique et leurs mélanges.

7. Procédé selon la revendication 1, **caractérisé en ce que** ledit acide est choisi parmi les échangeur d'ions inorganiques ou polymères à caractère acide ou les oxydes de métal inorganiques à caractère acide.

8. Procédé selon l'une des revendications précédentes, ledit acide étant mis en oeuvre dans une quantité comprise entre 0,01 et 10 mmol par g de mono- et/ou disaccharide.

9. Procédé selon l'une des revendications précédentes, ledit traitement mécanique étant un broyage qui consiste à réduire la granulométrie des matières à broyer en utilisant des corps de broyage.

10. Procédé selon l'une des revendications précédentes, le broyeur étant choisi parmi les broyeurs vibrants, les broyeurs à agitation, les broyeur à billes équipés d'un agitateur et les broyeurs à billes.

11. Procédé selon l'une des revendications précédentes, dans lequel le mélange contenant les produits de la réaction est débarrassé, suite audit traitement mécanique, de l'acide y adhérant puis, éventuellement, soumis à une séparation permettant d'obtenir les différents produits de la réaction.
